Europäisches Patentamt

European Patent Office

Office européen des brevets

(11) Publication number: **0 192 320**
A1

## (12) EUROPEAN PATENT APPLICATION

(21) Application number: 86300169.9

(22) Date of filing: 13.01.86

(51) Int. Cl.⁴: **C 12 Q 1/00**
G 01 N 33/543, G 01 N 33/531
C 12 N 9/96

(30) Priority: 11.01.85 GB 8500698

(43) Date of publication of application:
27.08.86 Bulletin 86/35

(84) Designated Contracting States:
AT BE CH DE FR GB IT LI NL SE

(71) Applicant: UNILEVER PLC
Unilever House Blackfriars P.O. Box 68
London EC4P 4BQ(GB)

(84) Designated Contracting States:
GB

(71) Applicant: UNILEVER NV
Burgemeester s'Jacobplein 1 P.O. Box 760
NL-3000 DK Rotterdam(NL)

(84) Designated Contracting States:
BE CH DE FR IT LI NL SE AT

(72) Inventor: Drave, Rosemary Ann Lucy
Pump Cottage 62 Main Street
Holcot Northampton NN6 9SP(GB)

(72) Inventor: Whiteley, Susan Catherine
35 Curlew Crescent
Brickhill Bedford MK41 7HX(GB)

(74) Representative: Stancliffe, Terence Christopher et al,
UNILEVER PLC Patents Division P.O. Box 68 Unilever
House
London EC4P 4BQ(GB)

(54) Preparation of reagents.

(57) Improved dried preparation of enzymatically active reagent material suitable for use as a reagent in a test procedure requiring said enzymatically active material, said preparation comprising a dried layer incorporating said enzymatically active material together with a carrier or binder material suitable to release said enzymatically active material upon contact with an aqueous reaction liquid: wherein said dried layer comprises, together with said enzymatically active material, a stabilising amount of a protein which is inert with respect to the test reaction in which said enzymatically active material is to participate, and optionally a layer-forming quantity of a sugar or sugar derivative. The dried preparations are generally carried on inert solid substrates, e.g. water impermeable and insoluble substrates such as plastics or glass.

EP 0 192 320 A1

Croydon Printing Company Ltd.

PREPARATION OF REAGENTS

This invention relates to the preparation and use of reagents, especially for example the preparation and use of dried forms of enzymatically active reagents, e.g. reagents suitable for enzyme-linked specific binding assays such as ELISA assays, using enzymes conjugated to antigens, antibodies or other specific binding materials. The invention also relates to reagent kits including such dried preparations.

The prior art includes many dried forms of biologically active reagents including serological reagents such as antibodies, enzymes and their coupling products. For example, USP 4 234 316 (Hevey), in describing devices for delivering precise measured quantities of a plurality of water-soluble reagents, carried on impervious supports, mentions in particular devices for carrying out radioimmunoassay for T4 comprising plastic carriers carrying film dried down from a solution of 20% dextran (70,000 m.w.) containing concentrated antiserum (anti-T4) for use as one of the reagent components of the radioimmunoassay, and other plastic carriers carrying film containing T4 standards in human serum. Also mentioned in USP 4 234 316 are reagents for clinical tests comprising carrier-based films containing inter alia enzymes, enzyme substrates, antibodies, antigens, haptens, and reagents carrying radioisotopic, fluorescent, enzyme, or cofactor tags. Binders are disclosed for use in such films, selected from polymeric materials such as inter alia dextran, acrylic gels and various gums, together with humectants, surface-active agents and low-m.w. substances such as sorbitol, tartrate, mannose and sucrose.

It is also known to use sugars in the preparation of dried forms of reagent for immunological tests, e.g. in the preparation of immunoadsorbents, as in GB 2 016 687 (Abbott).

Our experience in the use of compositions analogous to those of the prior art has not been entirely satisfactory: thus, although it is clear that they do give dried reagents with a degree of stability and preserved activity, we find that they are often considerably less effective in preserving and stabilising enzymatically active material, such as enzyme conjugates, than they are in preserving the antibody activity of immunoglobulin.

For example, in one test, a sugar preparation which preserved near 100% of an antibody activity only left 30% of an enzymic preparation in an active state. For some purposes such a degree of stabilisation may be adequate, but better preservative techniques are needed for some tests in which the quantity of enzyme activity is directly related to a quantitative or semiquantitative test result.

It is an aim of this invention to provide dried preparations of enzymatically active reagent materials which can yield improved preservation and stability.

It is another aim of this invention to provide dried preparations of enzymatically active materials which can quickly release their content into solution or dispersion upon contact with aqueous reaction media.

It is a further aim of the invention to provide dried preparations of enzymatically active materials which can give satisfactory consistency of preservation of their enzymatic activity on storage, e.g. without refrigeration and even under tropical conditions.

According to the invention there is provided, in a dried preparation of enzymatically active reagent material suitable for use as a reagent in a test procedure requiring said enzymatically active material, said preparation comprising a dried layer incorporating said enzymatically active material together with a carrier or binder material suitable to release said enzymatically active material upon contact with an aqueous reaction liquid, the improvement whereby said dried layer comprises, together with said enzymatically active material, a stabilising amount of a protein which is inert with respect to the test reaction in which said enzymatically active material is to participate, and optionally a layer-forming quantity of a sugar or sugar derivative.

The dried preparations are generally carried on inert solid substrates, e.g. water impermeable and insoluble substrates such as plastics or glass as more particularly described hereinbelow.

According to one form of the invention, we have found it possible to provide a convenient and relatively stable dry preparation of an enzyme which has been covalently coupled to other material, e.g. an antibody, to form a conjugate, comprising a dried amount of the conjugate, together with an inert protein indifferent to the assay reaction(s) in which the conjugate is to take part, and a sugar or sugar derivative, preferably dried from a buffered salt solution. The preparation quickly releases the conjugate upon rehydration.

The invention extends to the corresponding processes of producing such dried preparations, and to the use of such preparations as reagents in test methods such as specific binding assays, e.g. enzyme-linked immunoassays.

The invention also extends to test kits for such assays which comprise such dried preparations, for example a test kit for an immunoassay comprising an enzyme conjugated with a member of a specific binding pair relevant to the assay, in dried form as described herein, together with other reagents including another member of said specific binding pair and a substrate for said enzyme.

Sugars and sugar derivatives useful in connexion with these preparations include saccharides and saccharide alcohols, e.g. lactose, maltodextrin, sucrose or trehalose. These can be used in for example the form of solutions for drying down, suitably at up to about 20% w/v concentration, e.g. within the range of about 5% - 10% w/v. We have found that lactose and trehalose have been associated with best results in certain tests such as those detailed below.

Suitable examples of enzymatically active reagent materials which can be stabilised in dry form according to the present invention include for example a conjugate comprising an immunoglobulin with antibody specificity chemically coupled to an enzyme, such as for example alkaline phosphatase. Such a conjugate can be made by any of a number of well-known methods not constituting the present invention, e.g. by glutaraldehyde coupling technique.

Other examples of enzymes which can form part of conjugates to be stabilised by the means described herein are peroxidase, e.g. horseradish peroxidase, glucose oxidase, and catalase among others.

Suitable examples of inert proteins include albumins, e.g. bovine serum albumin or ovalbumin, and especially reagent grade preparations of such albumins. These can be used preferably for example at a concentration in the liquid which is to be dried down within the range of about 0.5% w/v (conveniently for example about 1% w/v) up to about 6% w/v, e.g. within the range of about 0.5% to 3%, or >=3% to <=6%. In certain test examples made with sucrose, major improvement in stabilised enzyme activity was achieved by including 1% w/v reagent grade ovalbumin, with lesser improvement arising with larger amounts of the inert protein up to about 4% w/v.

Higher concentrations of the protein and the sugar or sugar derivative than those quoted are often operable but sometimes give no additional benefit.

It can be very desirable also to include in the dried preparations a further material such as a salt and/or buffer, especially a further material which contains an ion or cofactor corresponding to the enzyme activity to be preserved, and functioning as an adjuvant of the preservative or otherwise.  For example, where a phosphatase is the enzymatic activity of interest, it is desirable to include a magnesium salt in the dried preparation.  A useful example of such a material is magnesium chloride-tris HCl, pH 8, especially in the case of alkaline phosphatase as the material to be stabilised. Salt and buffer concentrations can be chosen within a wide range at will.

Optional and desirable further ingredients are viscosity additives such as polyethyleneglycol, e.g. PEG 6000 at about for example 1.5% w/v, and detergents such as Triton, e.g. Triton-QS9 at about 0.01% w/v (Trade Mark).  Prior art other additives for dry preparations may also be present.

Up to 30% polyethyleneglycol and 0.2% Triton have been found usable and to lack deleterious effect on the preservation of the enzyme and antibody activity, besides possibly conferring advantage in the subsequent use of the reagent.

The substrate for the drying can be glass (e.g. a borosilicate glass tube), or plastics such as polystyrene (e.g. wells of a microtitre plate) or nylon (such as the surface of a stick, bead, strip or peg).  Generally the substrate can have any shape or form that may be desired or convenient for the performance of a test or assay in which the enzymatically active material is to participate.

The thickness of the dried layer can be chosen according to convenience, and is not particularly critical: suitable thicknesses have been found for example in the range up to about 50 microns, and sometimes up to about 0.1 mm. It can be convenient to apply the liquid to be dried to the surface of the solid substrate at a rate of for example within the order of about 0.2 - 2 microliter per square centimeter of surface to carry the active layer. Other rates of application however can well be chosen.

The drying process can be carried out by air-drying, vacuum-drying or lyophilisation, e.g. vacuum-drying after freezing in solid carbon dioxide at -80 deg. C. We find that air-drying in an oven at about for example 37 deg. C is a convenient method of drying and one that can show particularly well the stabilising advantage of the present invention.

Preparations made in this manner from a conjugate of alkaline phosphatase and anti-beta-HCG antibody have been found useful in the immunoassay of HCG based on a sandwich test technique using immunosorbent anti-HCG (well-known per se). The dried preparation releases the conjugate that it contains into an aqueous assay reaction medium rapidly, for example within about a minute, on contact between the preparation and the liquid.

We have found >85% retention of activity in storage trials lasting many weeks at 37 deg. C, in preparations of this kind.

Further details are given in the following illustrative example, without limitation of the invention thereto.

## Example

Dried reagent preparations of enzyme-antibody conjugate such as for example alkaline phosphatase and anti-HCG (both obtainable from commercial sources and coupled using standard glutaraldehyde technique) can be made with good preservation of both enzymatic and antibody activity and quick releasability upon exposure to aqueous reaction liquid for example as follows.

Carrier substrates are chosen for example from (chemically clean) borosilicate glass, polycarbonate plastics, polystyrene and nylon in the form of slides, strips, tubes and microtitre wells.

Suitable proportions (w/v) by way of example for ingredients in aqueous liquid to be used for drying down to produce a dried preparation in accordance with embodiments of the invention are:

> 10% sugar (e.g. especially lactose or trehalose),
> 6% ovalbumin (commercial reagent grade),
> 1 mM (or other convenient concentration) of
> magnesium chloride with 50mM tris.HCl pH 8,

(i.e. neutral or slightly alkaline), and any desired activity level of the enzyme conjugate such as alkaline-phosphatase-anti-HCG to be dried down (e.g. 1 - 100 microgram/ml).

In a preferred variation of these proportions, the quantity of ovalbumin is reduced to the range of about 1% to 3% w/v, and the liquid composition also contains 1.5% w/v polyethyleneglycol 6000 and 0.01% w/v Triton QS9 (Trade Mark) detergent.

Aliquots of about 10 microlitre, per microtitre well or other carrier, of the above-mentioned liquid composition, are applied to the respective carrier and the result air-dried at 37 deg. C. The dried preparations are preferably maintained thereafter until required for reagent use within dry water-impermeable enclosures, e.g. sealed plastics or metal foil packs, optionally including desiccant. It has been found after storage trials that such preparations maintain a satisfactory level and consistency of activity after several weeks storage even at temperatures as high as 37 deg. C, when used in conjunction with a per se known enzyme-immunoassay technique using a nylon immunosorbent sensitised with anti-HCG antibody, and a 'sandwich' test technique resulting in a coloured product from a phosphatase substrate varying directly with the amount of HCG present in the sample to be assayed.

In a modification of the above-described example, further tests were carried out with dried preparations which were otherwise similar to those described above except that the conjugate was a conjugate prepared in known manner from alkaline phosphatase enzyme and pregnanediol. At elevated (37 deg. C) storage temperatures, equivalent to tropical distribution and storage of clinical test reagent kits, the quantity of activity found after storage has been less after a week's storage than in cooler-stored material kept at room temperature in temperate climate, but no further loss was encountered after several more weeks, and satisfactorily little extra variation in activity was found to have arisen after such storage in the

0192320

unfavourable conditions.

Many modifications and variations will be apparent to the skilled reader in the light of the foregoing description, which is intended to extend to all combinations and subcombinations of features mentioned hereinabove and in the appended claims.

## CLAIMS

1.    In a dried preparation of enzymatically active reagent material suitable for use as a reagent in a test procedure requiring said enzymatically active material, said preparation comprising an inert solid substrate carrying a dried layer incorporating said enzymatically active material together with a carrier or binder material suitable to release said enzymatically active material upon contact with an aqueous reaction liquid, the improvement whereby said dried layer comprises, together with said enzymatically active material, a stabilising amount of a protein which is inert with respect to the test reaction in which said enzymatically active material is to participate, and optionally a layer-forming quantity of a sugar or sugar derivative.

2.    A dried preparation suitable for use as a reagent in a specific binding assay, the preparation comprising a solid substrate carrying an enzyme covalently conjugated to an antigen, antibody or other specific binding material, an inert protein indifferent to the assay reactions for which the preparation is to be used, and a sugar or sugar derivative, dried in admixture on said solid substrate, said preparation allowing release of said conjugate upon contact with aqueous liquid.

3.    A preparation according to claim 2, in which the sugar or sugar derivative comprises lactose, maltodextrin, sucrose or trehalose.

4.    A preparation according to claim 2 or 3, in which the inert protein comprises ovalbumin or serum albumin.

P3016

5.   A preparation according to claim 2, 3 or 4, dried from an aqueous liquid with a sugar concentration in the range up to about 20% w/v, e.g. the range of >=5% to <=10%.

6.   A preparation according to claim 2, 3, 4 or 5, dried from an aqueous liquid with an inert protein concentration of at least 0.5% w/v, for example up to about 3%.

7.   A preparation according to any of claims 1 to 6, based on a conjugate of alkaline phosphatase, and dried from a solution containing magnesium chloride and buffer at neutral or slightly alkaline pH.

8.   A preparation according to any of claims 1 to 7, and further comprising a viscosity additive, humectant and/or antimicrobial preservative.

9.   A preparation according to any of claims 1 to 8, wherein said solid substrate carrying said film is enclosed within a water-impermeable sealed enclosure.

10.   A test kit for an immunoassay comprising an enzyme conjugated with a member of a specific binding pair relevant to the assay, in dried form according to any of claims 1 to 9, together with other reagents including another member of said specific binding pair and a substrate for said enzyme.

11.   A process for producing a dried preparation according to any of claims 1 to 9, which comprises drying down on an inert solid substrate a liquid mixture containing said enzymatically active material together with a carrier or binder material suitable to release said enzymatically active material upon contact with an aqueous reaction liquid, said process incorporating the improvement whereby said liquid mixture comprises, together with said enzymatically active material, a stabilising amount of a protein which is inert with respect to the test reaction in which said enzymatically active material is to participate, and optionally a layer-forming quantity of a sugar or sugar derivative.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | EP-A-0 140 489 (WAKO PURE CHEMICAL INDUSTRIES LTD.) * Whole document * | 1-8,11 | C 12 Q 1/00<br>G 01 N 33/543<br>G 01 N 33/531<br>C 12 N 9/96 |
| X | PATENTS ABSTRACTS OF JAPAN, vol. 9, no. 76 (P-346) [1799], 5th April 1985; & JP - A - 59 206 761 (IGAKU SEIBUTSUGAKU KENKYUSHO K.K.) 22-11-1984 * Whole abstract * | 1,2,4, 11 | |
| X | GB-A-2 049 700 (INSTITUT BIOORGANICHESKOI KHIMII IMENI MM) * Whole document * | 1-6 | |
| A | PATENTS ABSTRACTS OF JAPAN, vol. 7, no. 238 (P-231) [1383], 22nd October 1983; & JP - A - 58 123 459 (MITSUI TOATSU KAGAKU K.K.) 22-07-1983 * Whole document * | 2,4-6, 11 | **TECHNICAL FIELDS SEARCHED (Int. Cl.4)**<br><br>C 12 Q<br>G 01 N<br>C 12 N |
| A | GB-A-2 124 231 (CORNING GLASS WORKS) * Whole document * | 2,4,6, 11 | |
| A | EP-A-0 042 755 (UNILEVER N.V.) * Abstract; page 10, lines 11-18; page 14, lines 10-22 * | 1-3,5, 11 | |

-/-

The present search report has been drawn up for all claims

| Place of search | Date of completion of the search | Examiner |
|---|---|---|
| THE HAGUE | 25-04-1986 | GRIFFITH G. |

# European Patent Office

## EUROPEAN SEARCH REPORT

EP  86 30 0169

| | DOCUMENTS CONSIDERED TO BE RELEVANT | | Page  2 |
|---|---|---|---|
| Category | Citation of document with indication, where appropriate, of relevant passages | Relevant to claim | CLASSIFICATION OF THE APPLICATION (Int. Cl.4) |
| A | EP-A-0 037 110  (TAKEDA YAKUHIN KOGYO K.K.) * Claims 4,6,10 * --- | 2,10 | |
| D,A | GB-A-2 016 687  (ABBOTT LABORATORIES) --- | | |
| A | DE-A-3 040 005  (EICKEN CHEMICAL CO., LTD.) --- | | |
| A | PATENTS ABSTRACTS OF JAPAN, vol. 9, no. 79 (P-347) [1802], 9th April 1985; & JP - A - 59 210 366 (SUMITOMO KAGAKU KOGYO K.K.) 29-11-1984 --- | | |
| A | EP-A-0 017 414  (FMC CORP.) & US - A - 4 234 316 (Cat. D) ----- | | TECHNICAL FIELDS SEARCHED (Int. Cl.4) |

The present search report has been drawn up for all claims

| Place of search | Date of completion of the search | Examiner |
|---|---|---|
| THE HAGUE | 25-04-1986 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82